# EUROPEAN PATENT SPECIFICATION

(11) **EP 2 753 190 B1**
(45) Date of publication and mention of the grant of the patent: **03.05.2017**
(21) Application number: 12759277.2
(22) Date of filing: 07.09.2012
(51) Int. Cl.: A23L 33/00, A23L 33/10, A61K 31/20, A61K 31/201

(54) **USE OF INFANT FORMULA WITH CHOLESTEROL**
VERWENDUNG VON SÄUGLINGSNAHRUNH MIT CHOLESTERIN
UTILISATIOND'UNE PREPARATION POUR NOURRISSONS CONTENANT DU CHOLESTEROL

(30) Priority: 08.09.2011 WO PCT/NL2011/050613
(43) Date of publication of application: 16.07.2014
(73) Proprietor: N.V. Nutricia, 2712 HM Zoetermeer (NL)
(72) Inventor: ABRAHAMSE-BERKEVELD, Marieke, NL-6666 HS Heteren (NL); VAN DER BEEK, Eline Marleen, NL-6705 DB Wageningen (NL); VAN DE HEIJNING, Hubertus Josephus Maria, NL-3523 PN Utrecht (NL)
(74) Representative: Nederlandsch Octrooibureau
(86) International application number: PCT/NL2012/050620
(87) International publication number: WO 2013/036119

(56) References cited:
- EP-A1- 1 800 675
- WO-A1-2010/027258
- WO-A1-2011/108934
- WO-A2-2007/073193

## Description

### FIELD OF THE INVENTION

The present invention is in the field of infant nutrition and especially in the field of later-in-life health effects of early-in-life nutrition.

### BACKGROUND OF THE INVENTION

Breast-feeding is the preferred method of feeding infants. However, there are circumstances that make breast-feeding impossible or less desirable. In those cases infant formulae are a good alternative. The composition of modern infant formulae is adapted in such a way that it meets many of the special nutritional requirements of the fast growing and developing infant. Still it seems that improvements can be made towards the constitution of infant milk formulae.

Human milk typically has a cholesterol content of 0.34 to 1.3 wt% based on total lipids (Jensen, 1999, Lipids 34, 1243-1271). Standard infant formula on the other hand do not comprise cholesterol. As a result, breast fed infants have higher blood cholesterol levels than bottle fed infants as long as the breast feeding period persists (Kallio et al, 1992, Paediatrics P683-666). However, later in life it appears that subjects who were breast fed early in life, have lower blood cholesterol levels compared to bottle fed infants during adulthood (Marmot, 1980, J Epid. Comm Health 34:164-167; Owen, 2006 Pediatrics 110:597-608). This may explain the increased risk on atherosclerosis and related vascular diseases later in life in subjects who were bottle fed compared to breast fed.
Non communicable diseases (NCD) like cardiovascular disease, including atherosclerosis, heart disease and high blood pressure are responsible for 35 million deaths/year today, which amounts to 60% of all deaths globally. Irrespective of income level, high cholesterol level is a top-10 leading risk factor for death. Yet a large contribution to the increase in NCDs is made by low-and middle income countries, especially as they undergo socio-economic improvement following reductions in infectious disease. The WHO projects an increase of 17% in NCDs over next decade globally.

The present invention relates to such future health effects of infant formulae, in particular to blood cholesterol levels and vascular diseases later in life.

EP 2305049 relates to a composition to be administered to an infant with the age below 36 months said composition comprising lipid, protein, digestible carbohydrate and cholesterol for use in the prevention of cardiovascular disease, atherosclerosis, and/or high blood cholesterol levels later in life.
WO 2008/054192 discloses the use of IMF with a specific fatty acid profile for preventing dislipidaemia later in life. WO 2007/073194 relates to infant formulae comprising phospholipids, sphingolipids, and cholesterol for the prevention of obesity later in life. WO 2009/138680 discloses the use of milk fat for infant nutrition for use in several purposes including regulation of blood cholesterol. WO 2010/027258 discloses infant compositions comprising vegetable lipid globules, having a volume weighted mode diameter above 1.0 µm, for the prevention of obesity later in life. However, these documents do not address the role of the location of the dietary cholesterol early in life on the reducing effects on blood cholesterol later in life.

### SUMMARY OF THE INVENTION

The inventors employed an animal model which is representative of the physiological programming effects of the diet taken early in life. In this model animals are fed with different diets early in life and subsequently they are exposed to the same Western style, high fat, high cholesterol diet. This model allows to examine how the early-in-life diet, via body programming effects, have a health effect later in life and how the early-in-life diet influences the way the body handles a Western style, high fat and cholesterol rich diet later on.

The inventors surprisingly found that the level of total cholesterol in the blood later in life after exposure to a Western style, high fat and cholesterol rich diet, was dependent on the way cholesterol was positioned in the early-in-life diet. When dietary cholesterol was located in the phospholipid layer coating the dietary lipid globules, total blood cholesterol later in life was advantageously decreased. When, on the other hand, dietary cholesterol was not present in the early-in-life diet in the coating of the lipid globules, the effect was reversed and total blood cholesterol later in life was increased. The decreasing effect on blood cholesterol levels later in life was further enhanced when the coated lipid globules were larger in size then typically present in infant formulae. Strikingly, these effects were already observed using dietary cholesterol concentrations much lower than present in human milk. A direct correlation of blood cholesterol levels later in life and obesity or relative amount of fat mass later in life was not observed. A direct diet effect on reducing blood cholesterol levels was not observed, instead a slight increase was observed.

Therefore the present invention relates to a nutritional composition comprising lipid, wherein the lipid is present in the form of lipid globules, said lipid globules comprising a core comprising triglycerides and a coating comprising phospholipids and cholesterol for use in feeding humans early in life for use in preventing hypercholesterolaemia and/or hyperlipoproteinemia, preventing artherosclerosis or other vascular diseases, reducing blood cholesterol levels, blood LDL (low-density lipoprotein) and/or blood VLDL (very low-density lipoprotein), later in life.

### DETAILED DESCRIPTION OF THE INVENTION

The present invention concerns a non-medical method for
a) reducing blood cholesterol levels, and/or
b) reducing levels of blood LDL and/or VLDL,
in a human subject when said human subject has reached an age above 36 months,
by feeding a nutritional composition comprising lipid, wherein the lipid is present in the form of lipid globules, said lipid globules comprising a core comprising triglycerides derived from vegetable origin and a surface layer comprising cholesterol and phospholipids to the human subject having an age of 0 to 36 months.

The invention also concerns a nutritional composition comprising lipid, wherein the lipid is present in the form of lipid globules, said lipid globules comprising a core comprising triglycerides derived from vegetable origin and a surface layer comprising cholesterol and phospholipids for use in feeding a human subject having an age of 0 to 36 months, for use in
a) reducing blood cholesterol levels, and/or
b) reducing levels of blood LDL and/or VLDL,
when said human subject has reached an age above 36 months.

The invention also concerns a nutritional composition comprising lipid, wherein the lipid is present in the form of lipid globules, said lipid globules comprising a core comprising triglycerides derived from vegetable origin and a surface layer comprising cholesterol and phospholipids, for use in feeding a human subject early in life, for use in
a) preventing hypercholesterolaemia and/or hyperlipoproteinemia, and/or
b) preventing atherosclerosis,
when said human subject has reached an age above 36 months.

The lipid in the nutritional compositions in the method or use according to the present invention is present in the form of lipid globules with a core comprising triglycerides derived from vegetable origin, preferably vegetable lipids, and a surface layer comprising cholesterol and phospholipids. Preferably essentially all the lipid in the nutritional compositions in the method or use according to the present invention is present in the form of lipid globules with a core comprising said triglycerides derived from vegetable origin and a coating comprising said cholesterol and phospholipids.

In the context of this invention, a human subject having an age of 0 to 36 months is also referred to as an infant.

### Lipid component

The present nutritional composition comprises lipid. The lipid provides preferably 30 to 60% of the total calories of the composition. More preferably the present composition comprises lipid providing 35 to 55% of the total calories, even more preferably the present composition comprises lipid providing 40 to 50% of the total calories. When in liquid form, e.g. as a ready-to-feed liquid, the composition preferably comprises 2.1 to 6.5 g lipid per 100 ml, more preferably 3.0 to 4.0 g per 100 ml. Based on dry weight the present composition preferably comprises 10 to 50 wt.%, more preferably 12.5 to 40 wt.% lipid, even more preferably 19 to 30 wt.% lipid.

Lipids include polar lipids (such as phospholipids, glycolipids, sphingomyelin, and cholesterol), monoglycerides, diglycerides, triglycerides and free fatty acids. Preferably the composition comprises at least 75 wt. %, more preferably at least 85 wt.% triglycerides based on total lipids.

The lipid of the present invention comprises vegetable lipids. The presence of vegetable lipids advantageously enables an optimal fatty acid profile, high in (poly)unsaturated fatty acids and/or more reminiscent to human milk fat. Using lipids from cow's milk alone, or other domestic mammals, does not provide an optimal fatty acid profile. This less optimal fatty acid profile, such as a large amount of saturated fatty acids, is known to result in increased cholesterol levels. Preferably the present composition comprises at least one, preferably at least two lipid sources selected from the group consisting of linseed oil (flaxseed oil), rape seed oil (such as colza oil, low erucic acid rape seed oil and canola oil), sunflower oil, high oleic sunflower oil, safflower oil, high oleic safflower oil, olive oil, coconut oil, palm oil and palm kernel oil. Preferably the present composition comprises at least one, preferably at least two lipid sources selected from the group consisting of linseed oil, canola oil, coconut oil, sunflower oil and high oleic sunflower oil. Commercially available vegetable lipids are typically offered in the form a continuous oil phase. When in liquid form, e.g. as a ready-to-feed liquid, the composition preferably comprises 2.1 to 6.5 g vegetable lipid per 100 ml, more preferably 3.0 to 4.0 g per 100 ml. Based on dry weight the present composition preferably comprises 10 to 50 wt.%, more preferably 12.5 to 40 wt.% vegetable lipid, even more preferably 19 to 30 wt.%. Preferably the composition comprises 45 to 100 wt.% vegetable lipids based on total lipids, more preferably 70 to 100 wt.%, even more preferably 75 to 97 wt.%. It is noted therefore that the present composition also may comprise non-vegetable lipids. Suitable non vegetable lipids include marine oils, microbial oils, egg fat and milk fat. Suitable and preferred non-vegetable lipids are further specified below.

### Phospholipids

The nutritional composition according to the present invention comprises phospholipids. Phospholipids are essential to form the surface layer, coating, of the lipid globules in which the dietary cholesterol can be located. Phosholipids are polar lipids. Polar lipids are amphipathic of nature and include glycerophospholipids, glycosphingolipids, sphingomyelin and/or cholesterol. Phospholipids refers to the sum of glycerophospholipids and sphingomyelin. According to the present invention the phospholipids are present as a coating of the lipid globule. By 'coating' is meant that the outer surface layer of the lipid globule comprises polar lipids, in particular phospholipids, whereas these polar lipids are virtually absent in the core of the lipid globule. The presence of phospholipids as a coating or outer layer of the lipid globule in the diet administered early in life enables the cholesterol to be located in the surface layer and this was found to advantageously reduce the blood cholesterol levels later in life.

The present composition preferably comprises glycerophospholipids. Glycerophospholipids are a class of lipids formed from fatty acids esterified at the hydroxyl groups on carbon-1 and carbon-2 of the backbone glycerol moiety and a negatively-charged phosphate group attached to carbon-3 of the glycerol via an ester bond, and optionally a choline group (in case of phosphatidylcholine, PC), a serine group (in case of phosphatidylserine, PS), an ethanolamine group (in case of phosphatidylethanolamine, PE), an inositol group (in case of phosphatidylinositol, PI) or a glycerol group (in case of phosphatidylglycerol, PG) attached to the phosphate group. Lysophospholipids are a class of phospholipids with one fatty acyl chain. Preferably the present composition contains PC, PS, PI and/or PE, more preferably at least PC.

The present composition preferably comprises sphingomyelin. Sphingomyelins have a phosphorylcholine or phosphorylethanolamine molecule esterified to the 1-hydroxy group of a ceramide. They are classified as phospholipid as well as sphingolipid, but are not classified as a glycerophospholipid nor as a glycosphingolipid. Sphingomyelin is especially preferred, since it interacts with cholesterol. Preferably the phospholipids are derived from milk lipids. A preferred source for phospholipids, particularly PC, is soy lecithin and/or sunflower lecithin.

The present composition preferably further comprises glycosphingolipids. The term glycosphingolipids as in the present invention particularly refers to glycolipids with an amino alcohol sphingosine. The sphingosine backbone is O-linked to a charged headgroup such as ethanolamine, serine or choline backbone. The backbone is also amide linked to a fatty acyl group. Glycosphingolipids are ceramides with one or more sugar residues joined in a β-glycosidic linkage at the 1-hydroxyl position. Preferably the present composition contains gangliosides, more preferably at least one ganglioside selected from the group consisting of GM3 and GD3.
Preferably the weight ratio of phospholipids : glycosphingolipids is from 2:1 to 10:1, more preferably 2:1 to 5:1.

Preferably the present composition comprises 0.5 to 20 wt.% phospholipids based on total lipid, more preferably 0.5 to 10 wt.%, more preferably 1 to 10 wt.%, even more preferably 2 to 10 wt.% even more preferably 3 to 8 wt.% phospholipids based on total lipid.
Preferably the present composition comprises at least 5 wt.% sphingomyelin based on total phospholipids, more preferably at least 10 wt.%, even more preferably at least 15 wt.%, preferably no more than 60 wt.%.

### Dietary cholesterol

The present composition comprises cholesterol. The present composition preferably comprises at least 0.02 wt.% cholesterol based on total lipid, more preferably at least 0.03 wt.%, more preferably at least 0.04 wt.%, even more preferably at least 0.1 wt.% based on total lipid. Since the cholesterol level lowering effects later in life were already observed at low cholesterol levels, preferably the amount of cholesterol does not exceed 5 wt.% based on total lipid, more preferably does not exceed 2 wt.%, more preferably does not exceed 1 wt.% of total lipid, even more preferably does not exceed 0.3 wt% based on total lipid.

Preferred sources for providing the phospholipids and/or cholesterol are egg lipids, milk fat, buttermilk fat and butter serum fat (such as beta serum fat). Preferably the phospholipid and/or cholesterol are derived from cow's milk. Preferably the phospholipid and cholesterol are from the same source. This will enhance the presence of dietary cholesterol in a layer of phospholipid in the final nutritional product. Derived from milk includes milk lipid, cream lipid, butter serum lipid, whey lipid, cheese lipid and/or buttermilk lipid and isolated therefore. The buttermilk lipid is typically obtained during the manufacture of buttermilk. The butter serum lipid or beta serum lipid is typically obtained during the manufacture of anhydrous milk fat from butter. Preferably the phospholipids, glycosphingolipids and/or cholesterol are obtained from milk cream. The composition preferably comprises phospholipids, glycosphingolipids and/or cholesterol from milk of cows, mares, sheep, goats, buffalos, horses and camels. It is most preferred to use a lipid extract isolated from cow's milk. The use of phospholipids from milk fat advantageously comprises the milk fat globule membranes, which are more reminiscent to the situation in human milk. The concomitant use of phospholipids derived from domestic animals milk and trigycerides derived from vegetable lipids therefore enables to manufacture coated lipid globules with a coating more similar to human milk, while at the same time providing an optimal fatty acid profile. Suitable commercially available sources for milk phospholipids, including sphingomyelin, and/or cholesterol are BAEF, SM2, SM3 and SM4 powder of Corman, Salibra of Glanbia, and LacProdan MFGM-10 or PL20 from Arla. Preferably the source of milk phospholipids comprises at least 4 wt.% phospholipids based on total lipid, more preferably 7 to 75 wt.%, most preferably 20 to 70 wt.% phospholipids based on total lipid. Preferably the weight ratio phospholipids to protein is above 0.10, more preferably above 0.20, even more preferably above 0.3. Preferably at least 25 wt.%, more preferably at least 40 wt.%, most preferably at least 75 wt.% of the phospholipids is derived from milk phospholipids.

### Fatty acid composition

Herein LA refers to linoleic acid and/or acyl chain (18:2 n6); ALA refers to α-linolenic acid and/or acyl chain (18:3 n3); LC-PUFA refers to long chain polyunsaturated fatty acids and/or acyl chains comprising at least 20 carbon atoms in the fatty acyl chain and with 2 or more unsaturated bonds; DHA refers to docosahexaenoic acid and/or acyl chain (22:6, n3); EPA refers to eicosapentaenoic acid and/or acyl chain (20:5 n3); ARA refers to arachidonic acid and/or acyl chain (20:4 n6); DPA refers to docosapentaenoic acid and/or acyl chain (22:5 n3). Medium chain fatty acids (MCFA) refer to fatty acids and/or acyl chains with a chain length of 6, 8 or 10 carbon atoms.

LA preferably is present in a sufficient amount in order to promote a healthy growth and development, yet in an amount as low as possible to prevent occurrence of obesity later in life. The composition therefore preferably comprises less than 15 wt.% LA based on total fatty acids, preferably of 5 to 14.5 wt.%, more preferably of 6 to 10 wt.%. Preferably the composition comprises over 5 wt.% LA based on fatty acids. Preferably ALA is present in a sufficient amount to promote a healthy growth and development of the infant. The present composition therefore preferably comprises at least 1.0 wt.% ALA based on total fatty acids. Preferably the composition comprises at least 1.5 wt.% ALA based on total fatty acids, more preferably at least 2.0 wt.%. Preferably the composition comprises less than 10 wt.% ALA, more preferably less than 5.0 wt.% based on total fatty acids. The weight ratio LA/ALA should be well balanced in order to prevent obesity and hypertriglyceridaemia later in life, while at the same time ensuring a normal growth and development. Therefore, the present composition preferably comprises a weight ratio of LA/ALA of 2 to 15, more preferably of 2 to 7, more preferably of 4 to 7, more preferably of 3 to 6, even more preferably of 4 to 5.5, even more preferably of 4 to 5.

Since MCFA contribute to a reduced blood cholesterol level, the present composition preferably comprises at least 3 wt.% MCFA based on total fatty acids, more preferably at least 10 wt.%, even more preferably at least 15 wt.%. Preferably, the present composition advantageously comprises less than 50 wt.% MCFA based on total fatty acids, more preferably less than 40 wt.%, even more preferably less than 25 wt.%.

Preferably the present composition comprises n-3 LC-PUFA, since n-3 LC-PUFA have a beneficial effect on cardiovascular disease risk and vascular fatty acid membrane composition. More preferably, the present composition comprises EPA, DPA and/or DHA, even more preferably DHA. Since a low concentration of DHA, DPA and/or EPA is already effective and normal growth and development are important, the content of n-3 LC-PUFA in the present composition, preferably does not exceed 15 wt.% of the total fatty acid content, preferably does not exceed 10 wt.%, even more preferably does not exceed 5 wt.%. Preferably the present composition comprises at least 0.2 wt.%, preferably at least 0.5 wt.%, more preferably at least 0.75 wt.% n-3 LC-PUFA based on total fatty acid content. Preferably the present composition comprises at least 0.2 wt.%, preferably at least 0.5 wt.%, more preferably at least 0.75 wt.% of the sum of DHA, DPA and EPA based on total fatty acid content.

As the group of n-6 fatty acids, especially arachidonic acid (AA) and LA as its precursor, counteracts the group of n-3 fatty acids, especially DHA and EPA and ALA as their precursor, the present composition comprises relatively low amounts of AA. The n-6 LC-PUFA content preferably does not exceed 5 wt.%, more preferably does not exceed 2.0 wt.%, more preferably does not exceed 0.75 wt.%, even more preferably does not exceed 0.5 wt.%, based on total fatty acids. Since AA is important in infants for optimal functional membranes, especially membranes of neurological tissues, the amount of n-6 LC-PUFA is preferably at least 0.02 wt.% more preferably at least 0.05 wt.%, more preferably at least 0.1 wt.% based on total fatty acids, more preferably at least 0.2 wt.%. The presence of AA is advantageous in a composition low in LA since it remedies LA deficiency. The presence of, preferably low amounts, of AA is beneficial in nutrition to be administered to infants below the age of 6 months, since for these infants the infant formulae is generally the only source of nutrition.

Preferably in addition to the vegetable lipid, a lipid selected from fish oil (preferably tuna fish oil) and single cell oil (such as algal, microbial oil and fungal oil) is present. These sources of oil are suitable as LC-PUFA sources. Preferably as a source of n-3 LC-PUFA single cell oil, including algal oil and microbial oil, is used, since these oil sources have a low EPA/DHA ratio.. Thus in one embodiment the present composition further comprises at least one lipid selected from the group consisting of fish oil, marine oil, algal oil, fungal oil and microbial oil.

### Lipid globule design

### Coating with phospholipids and cholesterol

The nutritional composition for use according to the present invention comprises comprises phospholipids and cholesterol. Phospholipids are present as a coating on the surface of the lipid globule. By 'coating' is meant that the outer surface layer of the lipid globule comprises polar lipids, whereas these polar lipids are virtually absent in the core of the lipid globule. Not all phospholipids and/or glycolipids that are present in the composition need necessarily be comprised in the coating, but preferably a major part is. Preferably more than 50 wt.%, more preferably more than 70 wt. %, even more preferably more than 85 wt.%, most preferably more than 95 wt.% of the phospholipids and/or glycolipids that are present in the composition are comprised in the coating of lipid globules. The phospholipids are located on the surface of the lipid globule, i.e. as a coating or outer layer. The presence of the phospholipids in the coating accommodates the cholesterol in order for it to be located in the surface layer as well. A suitable way to determine whether the phospholipids are located on the surface of the lipid globules is laser scanning microscopy as given in WO 2010/0027259.

In standard infant formulae, due to the small size of the lipid globule (resulting in a high lipid surface) and the very low levels of phospholipids present (typically below 0.15 wt% based on total fat), the lipid globules are not coated with phospholipids, but are covered for the main part by protein, such as casein.

### Lipid globule size

According to the present invention, lipid is present in the composition in the form of lipid globules. When in liquid form these lipid globules are emulsified in the aqueous phase. Alternatively the lipid globules are present in a powder and the powder is suitable for reconstitution with water or another food grade aqueous phase. The lipid globules comprise a core and a surface. The core preferably comprises vegetable fat and preferably comprises at least 90 wt.% triglycerides and more preferably essentially consists of triglycerides. Not all vegetable lipids that are present in the composition need necessarily be comprised in the core of lipid globules, but preferably a major part is, preferably more than 50% wt.%, more preferably more than 70 wt.%, even more preferably more than 85 wt.%, even more preferably more than 95 wt.%, most preferably more than 98 wt.% of the vegetable lipids that are present in the composition are comprised in the core of lipid globules. In one embodiment the core of the lipid globules comprises at least 70 wt.% triglycerides of vegetable origin, more preferably the core of the lipid globules comprises at least 85 wt.%, more preferably at least 95 wt.% triglycerides of vegetable origin.
In one embodiment the lipid globules of the present invention preferably have a volume-weighted mode diameter above 1.0 µm, preferably above 3.0 µm, more preferably 4.0 µm or above, preferably between 1.0 and 10 µm, more preferably between 2.0 and 8.0 µm, even more preferably between 3.0 and 8.0 µm, most preferably between 4.0 µm and 8.0 µm. Preferably in addition the size distribution is in such a way that at least 45 volume %, preferably at least 55 volume %, even more preferably at least 65 volume %, even more preferably at least 75 volume % has a diameter between 2 and 12 µm. More preferably at least 45 volume %, preferably at least 55 volume %, even more preferably at least 65 volume %, even more preferably at least 75 volume % has a diameter between 2 and 10 µm. Even more preferably at least 45 volume %, preferably at least 55 volume %, even more preferably at least 65 volume %, even more preferably at least 75 volume % has a diameter between 4 and 10 µm. It was found that coated large lipid globules have an improved effect on blood cholesterol levels later in life.
The percentage of lipid globules is based on volume of total lipid. The mode diameter relates to the diameter which is the most present based on volume of total lipid, or the peak value in a graphic representation, having on the X-as the diameter and on the Y-as the volume (%).

Standard infant formulae or growing up milks have lipid globules with mode diameter below 0.5 um.
The volume of the lipid globule and its size distribution can suitably be determined using a particle size analyzer such as a Mastersizer (Malvern Instruments, Malvern, UK), for example by the method described in Michalski et al, 2001, Lait 81: 787-796.

Methods for obtaining lipid globules with a coating of phospholipids, and increased size are disclosed in WO 2010/0027258 and WO 2010/0027259. The lipid globule size can be manipulated by adjusting process steps by which the present composition is manufactured. A suitable and preferred way to obtain lipid globules coated with phospholipids is to increase the amount of phospholipids compared to amounts typically present in infant formula and to have these phospholipids present during the homogenization process, wherein the mixture of aqueous phase and oil phase is homogenized. In standard infant milk formula the lipid fraction (usually comprising vegetable fat, a small amount of polar lipids and fat soluble vitamins) is mixed into the aqueous fraction (usually comprising water, skimmed milk, whey, digestible carbohydrates such as lactose, water soluble vitamins and minerals and optionally non-digestible carbohydrates) by homogenization under high pressure resulting in small lipid globules. If no homogenization was to take place, the lipid part would cream very quickly, i.e. separate from the aqueous part and collect at the top.

### Digestible carbohydrate component

The present nutritional composition preferably comprises digestible carbohydrate. The digestible carbohydrate preferably provides 30 to 80% of the total calories of the composition. Preferably the digestible carbohydrate provides 40 to 60% of the total calories. When in liquid form, e.g. as a ready-to-feed liquid, the composition preferably comprises 3.0 to 30 g digestible carbohydrate per 100 ml, more preferably 6.0 to 20, even more preferably 7.0 to 10.0 g per 100 ml. Based on dry weight the present composition preferably comprises 20 to 80 wt.%, more preferably 40 to 65 wt.% digestible carbohydrates.

Preferred digestible carbohydrate sources are lactose, glucose, sucrose, fructose, galactose, maltose, starch and maltodextrin. Lactose is the main digestible carbohydrate present in human milk. The present composition preferably comprises lactose. The present composition preferably comprises digestible carbohydrate, wherein at least 35 wt.%, more preferably at least 50 wt.%, more preferably at least 75 wt.%, even more preferably at least 90 wt.%, most preferably at least 95 wt.% of the digestible carbohydrate is lactose. Based on dry weight the present composition preferably comprises at least 25 wt.% lactose, preferably at least 40 wt.%.

### Non digestible carbohydrates

Preferably the present nutritional composition comprises non-digestible oligosaccharides. Preferably the present composition comprises non-digestible oligosaccharides with a degree of polymerization (DP) between 2 and 250, more preferably 3 and 60. The non-digestible oligosaccharides as dietary fiber may have a beneficial effect on reducing the risk on cardiovascular diseases.
Preferably the present nutritional composition comprises fructo-oligosaccharides, galacto-oligosaccharides and/or galacturonic acid oligosaccharides, more preferably galacto-oligosaccharides, most preferably transgalacto-oligosaccharides. In a preferred embodiment the composition comprises a mixture of transgalacto-oligosaccharides and fructo-oligosaccharides. Suitable non-digestible oligosaccharides are for example Vivinal GOS (Friesland Campina DOMO), Raftilin HP or Raftilose (Orafti). Transgalacto-oligosaccharide is preferred since it may increase insulin sensitivity.

Preferably, the nutritional composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 20 wt.%, more preferably 0.5 wt.% to 10 wt.%, even more preferably 1.5 wt.% to 7.5 wt.%. A lower amount of non-digestible oligosaccharides will be less effective in reducing cardiovascular disease risk, whereas a too high amount will result in side-effects of bloating and abdominal discomfort.

### Protein

The present nutritional composition preferably comprises proteins. The protein component preferably provides 5 to 15% of the total calories. Preferably the present composition comprises a protein component that provides 6 to 12% of the total calories. More preferably protein is present in the composition at most 9% based on calories, more preferably the composition comprises of 7.2 to 8.0% protein based on total calories, even more preferably of 7.3 to 7.7% based on total calories. Human milk comprises a lower amount of protein based on total calories than cow's milk. The protein concentration in a nutritional composition is determined by the sum of protein, peptides and free amino acids. Based on dry weight the composition preferably comprises at most 12 wt.% protein, more preferably of 9.6 to 12 wt.%, even more preferably 10 to 11 wt.%. Based on a ready-to-drink liquid product the composition preferably comprises at most 1.5 g protein per 100 ml, more preferably of 1.2 to 1.5 g, even more preferably of 1.25 to 1.35 g.

The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. In case whey proteins are used, the protein source is preferably based on acid whey or sweet whey, whey protein isolate or mixtures thereof and may include α-lactalbumin and β-lactoglobulin. More preferably, the protein source is based on acid whey or sweet whey from which caseino-glyco-macropeptide (CGMP) has been removed. Preferably the composition comprises at least 3 wt.% casein based on dry weight. Preferably the casein is intact and/or non-hydrolyzed. For the present invention protein includes peptides and free amino acids.

### Other

The present nutritional composition is preferably particularly suitable for providing the daily nutritional requirements to a human with an age below 36 months, particularly an infant with the age below 24 months, even more preferably an infant with the age below 18 months, most preferably below 12 months of age. Hence, the nutritional composition is for feeding or is used for feeding a human subject. The present composition preferably comprises lipid, and protein and digestible carbohydrate wherein the lipid preferably provides 30 to 60 % of total calories, the protein preferably provides 5 to 20 %, more preferably 5 to 15 wt.%, of the total calories and the digestible carbohydrate preferably provides 25 to 75% of the total calories. Preferably the present nutritional composition comprises lipid providing 35 to 50% of the total calories, protein providing 6 to 12 % of the total calories and digestible carbohydrate providing 40 to 60 % of the total calories. In one embodiment, the protein provides 5 to 9 % of the total calories. The amount of total calories is determined by the sum of calories derived from protein, lipids and digestible carbohydrates.

The present composition is not human breast milk. The present composition is not (raw) cow's or other (raw) mammalian milk. The present composition comprises vegetable lipids. The composition of the invention preferably comprises other ingredients, such as vitamins, minerals according to international directives for infant formulae.

In one embodiment, the nutritional composition according to the invention or the nutritional composition for use according to the invention is a preterm formula, infant formula, follow on formula or growing up milk.

In order to meet the caloric requirements of the infant, the composition preferably comprises 50 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/l, more preferably 260 to 320 mOsmol/l.

Preferably the composition is in a liquid form, with a viscosity below 35 mPa.s, more preferably below 6 mPa.s as measured in a Brookfield viscometer at 20°C at a shear rate of 100 s⁻¹. In one embodiment, the present composition is a powder. Suitably, the composition is in a powdered form, which can be reconstituted with water or other food grade aqueous liquid, to form a liquid, or in a liquid concentrate form that should be diluted with water. It was found that lipid globules maintained their size and coating when reconstituted.
When the composition is in a liquid form, the preferred volume administered on a daily basis is in the range of about 80 to 2500 ml, more preferably about 450 to 1000 ml per day.

### Application

The inventors surprisingly found that when mice during infancy and childhood were fed a food composition comprising lipid globules coated with phospholipids and cholesterol, a different and significant effect on blood cholesterol levels later in life was observed compared to mice which during infancy and childhood had been fed a food composition having a similar fatty acid composition, but no phospholipids and cholesterol present in the form of a coating around the dietary lipid globule. From day 42 both groups were fed a Western style diet which was high in fat and cholesterol. Surprisingly at day 98, which is a time point corresponding to adulthood in humans, the mice, which had previously consumed the food composition of the present invention before turning to the Western style diet, had a lower blood cholesterol level than mice which had received a control composition. The effects were even more pronounced when the lipid globules were increased in size. Phospholipids and cholesterol surprisingly had to be present as a coating around the lipid globules. If this was not the case the later in life effect on blood cholesterol levels was reversed. A similar experiment performed in rats, showed the same results and also indicated that the effects at day 42, directly after the intervention diet and a time point corresponding to (late) childhood in a human setting, had not yet these effects on blood cholesterol. The blood cholesterol lowering effect thus was mainly due to the effects occurring during exposure to the Western style diet, i.e. the way the metabolism deals with the Western style, high fat, cholesterol rich diet. A one to one correlation with fat mass or relative fat mass or visceral fat mass was not observed. This is also the case in a human setting, wherein sometimes obese subjects do not necessarily have hypercholesterolaemia and, on the other hand, non-obese subjects can suffer from hypercholesterolaemia. See for example Rigaud et al (2009) Diabetes Metab. 35:57-63 disclosing high cholesterol levels in anorexia nervosa patients. Also no one to one correlation with blood triglyceride levels was observed.

The present composition is administered to the human subject during the first 36 months of life. This period from birth up to childhood represents a critical timeframe during which nutrition programs the metabolism and body composition, still under development at this time, towards its function/setting later in life. The present composition is advantageously administered to a human of 0 to 24 months, more preferably to a human of 0 to 18 months, even more preferably to a human of 0 to 12 months, most preferably to a human of 0 to 6 months of age. The present invention particularly aims to reduce blood cholesterol levels later in life and is not a treatment to reduce existing high blood cholesterol levels. Preferably the composition is to be used in infants having a weight appropriate for gestational age.

The present composition is preferably administered orally to the infant. The present invention also aims to prevent hypercholesterolaemia and/or hyperlipoproteinaemia at the age above 36 months. Increased levels of blood cholesterol, hypercholesteroleamia and/or hyperlipoproteinaemia are an important risk factor and cause for vascular diseases. Blood cholesterol levels, levels of blood HDL, LDL or VLDL in the present invention also relate to plasma levels of cholesterol, HDL, LDL or VLDL. In other words, when it is stated that nutritional compositions have an effect on plasma cholesterol, HDL, LDL or VLDL levels, this includes effects on blood cholesterol, HDL, LDL or VLDL levels.
Therefore, in one embodiment the present method is for preventing vascular diseases selected from the group consisting of atherosclerosis, cardiovascular disease, cerebrovascular disease, ischaemia, peripheral vascular disease, and renal artery disease, in particular atherosclerosis, of a human subject when said human subject has an age above 36 months, preferably when said human subject has an age above 5 years, particularly above 13 years, more particularly above 17 years. The present invention also aims to improve cardiovascular function later in life. The present invention also aims to reduce blood cholesterol levels and/or to reduce levels of blood LDL and/or VLDL of a human subject when said human subject has an age above 36 months. In one embodiment the present method is for reducing blood cholesterol levels and/or reducing levels of blood LDL and/or VLDL of a human subject when said human subject has an age above 36 months, preferably when said human subject has an age above 5 years, particularly above 13 years, more particularly above 17 years. Reduction of blood cholesterol levels, VLDL and/or LDL levels means a reduced concentration compared to the concentrations found in subjects who were bottle fed with standard infant formula during infancy. In one embodiment reduction of blood cholesterol levels, VLDL and/or LDL levels means a reduced concentration compared to the concentrations found in subjects who were bottle fed with standard infant formula during infancy and more similar to the values found in subjects who were breast fed for at least 3 months.

With later in life is meant an age exceeding the age at which the diet is taken, preferably exceeding said age with at least one year.

Preterm and/or small for gestational age infants often encounter catch up growth early in life. This is generally seen as a risk factor for later in life high cholesterol levels and vascular diseases. So the composition of the present invention is advantageously used in preterm infants or small for gestational age (SGA) infants, in particular for feeding a preterm infant or an infant small for gestational age. A preterm or premature infant relates to an infant born before the standard period of pregnancy is completed before or on 37 weeks pregnancy of the mother, i.e. before or on 37 weeks from the beginning of the last menstrual period of the mother. SGA babies are those whose birth weight lies below the 10th percentile for that gestational age. Premature and/or SGA infants include low birth weight infants (LBW infants), very low birth weight infants (VLBW infants), and extremely low birth weight infants (ELBW infants). LBW infants are defined as infants with a weight less than 2500 g. VLBW infants as infants with a weight which is less than 1500 g, and ELBW infants as infants with a weight less than 1000 g.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Programming effect of lipid globule architecture on plasma cholesterol levels later in life

Infant milk formulae were prepared similar to example 1 B of WO 2010/0027258.
1) *a control IMF:* This diet comprises 255 g lipid per kg dry weight. Lipid globules were small. No polar lipids were added. Lipid comprised about 47 % digestible carbohydrates about 45 and protein about 8% of total calories.
2) *an IMF with added phospholipids and cholesterol:* This diet differed from diet 1 in that it comprised small lipid globules and dry blended added phospholipids (PL) and cholesterol derived from milk, i.e. the phospholipids not located at the outer surface layer of the lipid.
3) *an IMF of the present invention:* This diet differed from diet 1 in that the small lipid globules comprised coating with phospholipids and cholesterol derived from milk
4) *an IMF of the present invention:* This diet differed from diet 3 in that it comprised large lipid globules coated with phospholipids and cholesterol derived from milk.

Differences in lipid globule size were obtained by differences in homogenization pressure as described in example 1 of WO 2010/0027258. The pressure of homogenization was 10/5 for diet 4 and 550/50 for diet 1 to 3. The volume mode diameter of the lipid globules of diet 1 to 3 ranged from 0.23 to 0.28 µm. Less than 10 vol.% had a diameter between 2 and 12 µm. The volume mode diameter of the lipid globules in diet 4 was 3.0 µm. More than 50 vol.% had a diameter between 2 and 12 µm. The source of phospholipids for diet 2 to 4 was SM2 powder from Corman which was used at a final concentration of about 1.3 % phospholipid based on total lipid and about 0.045 wt% cholesterol based on total lipid. The amount of sphingomyelin was about 20 wt. % based on total phospholipids.

The fatty acid composition of the experimental diets was very similar in respect of saturated, mono-unsaturated, poly unsaturated and long chain poly unsaturated fatty acids, with calculated linoleic acid (LA) of about 14 wt.% based on total fatty acids, with alpha-linoleic acid (ALA) of about 2.6 wt.% based on total fatty acids and with LA/ALA weight ratio of about 5.4. The amount of DHA was 0.2 wt.% based on total fatty acids in all 4 diets, and the amount of ARA was about 0.35 wt.% based on total fatty acids. EPA and DPA levels were about 0.04 and 0.01 wt.% based on total fatty acids.

Subsequently rodent diets were prepared comprising 282 g IMF 1, 2, 3, or 4 per kg. The rest of the diet was AIN-93G protein, carbohydrates and fibre. All lipid (about 72 g per kg rodent diet) present in the diet was derived from the IMF

Offspring of C57/BL6 dams were weaned from day 15 on. The experimental weaning diets were continued until day 42 (n=12 per diet). From day 42 to day 98 all pups were fed the same diet based on AIN-93G diet with an adjusted lipid fraction (containing 20 wt.% lipid of which 50 wt.% lard and 1 % cholesterol, based on total lipid), comprising 4520 kJ per 100 g, 52 wt% digestible carbohydrates, 4.75 wt.% fibers, and 17.9 wt.% protein, which is representative for a Western style, high fat, high cholesterol diet. In the Western style diet the weight ratio LA/ALA was 9.15. No LC-PUFA were present.

At the age of 98 days a blood sample was taken and HDL (high-density lipoprotein), VLDL (very low-density lipoprotein), LDL (low-density lipoprotein), and total cholesterol levels were determined in blood plasma as known in the art. In short after fasting, blood samples were obtained under general anesthesia (isoflurane/N2O/O2) by cardiac puncture. Blood samples were collected in K3EDTA-coated 1 mL microtubes (Greiner Bio-one, Germany), centrifuged during 15 minutes at 4 °C (3500 rpm, Biofuge fresco, Heraeus) and plasma and erythrocytes samples were stored at -80 °C until further analysis. Plasma cholesterol levels were measured by colorimetric analysis after enzymatic hydrolysis of cholesterolesters (total) in presence of selective detergent (HDL) and after a precipitation (LDL) to obtain the several cholesterol fractions (Reinier de Graaf laboratory, Delft, The Netherlands). VLDL fraction was calculated from total, LDL and HDL cholesterol levels.
Body weight was measured and body composition (% fat mass) was determined by DEXA scan. Adipose tissue was collected and the sum of epididymal fat (EPI), retroperitoneal fat (RP) en perirenal fat (PR) was taken as the amount of visceral fat.

The results are shown in Table 1. Surprisingly the level of total cholesterol in the blood later in life was different when the cholesterol administered early in life was present in the diet in a different form. When present in the diet, but not present in the coating of the lipid globule, total blood cholesterol was increased. When present in the diet in the coating of the lipid globule the effect was reversed and total blood cholesterol later in life was advantageously decreased. This decreasing effect was even higher when the lipid globules were larger in size. The same effects were observed for HDL, LDL and VLDL with very little overall effect on the ratio of total cholesterol/HDL or LDL/HDL (data not shown). A decrease in total cholesterol, and LDL and VLDL is the most predictive parameter for decreased risk on atherosclerosis and other vascular diseases. Although mice consuming diet 4 had the lowest relative fat mass (relative to total body weight) later in life, no clear relationship between blood cholesterol levels and relative fat mass and visceral fat mass later in life was observed., as observed for example when comparing diet 3 and diet 4.

In an experiment performed with mice wherein direct effects diet effects of similar diets of example 1 were tested on day 42 only, it could be shown that cholesterol levels due to direct diet effects were somewhat higher in diet 4, and somewhat lower in diet 2 (data not shown).

**Table 1: Total Blood cholesterol levels (mmol/l) and fat mass (%) and visceral fat (g) of mice on day 98 exposed to different diets from day 15 to day 42.**

| Diet | | | cholesterol | Fat % | Visceral fat |
|---|---|---|---|---|---|
| 1 | Small, no PL | Mean | 3.57 | 23.10 | 1.43 |
| | | S.E. | 0.19 | 1.16 | 0.11 |
| 2 | Small, added PL | Mean | 3.99 | 25.17 | 1.57 |
| | | S.E. | 0.24 | 1.17 | 0.12 |
| 3 | Small, PL coating | Mean | 3.16 | 24.18 | 1.37 |
| | | S.E. | 0.34 | 1.81 | 0.19 |
| 4 | Large, PL coating | Mean | 3.10 | 19.77 | 1.14 |
| | | S.E. | 0.20 | 1.23 | 0.16 |

### Example 2: Programming effect of lipid globules architecture on plasma cholesterol levels later in life

The experiment of example 1 was repeated with diets similar to diet 1 and 4 of example 1. Instead of mice, male Wistar rats were used. Instead of day 98, blood was analyzed both at day 42 (direct diet effect) and at day 130 (later in life, programming effect).. The amount of phospholipid in diet 4 was 1.6 wt.% based on total fat (and 0.11 wt% in diet 1). The amount of cholesterol was 0.03 wt% in diet 4 on total fat. Linoleic acid and alpha-linoleinic acid of diet 1 and 4 were the same as for example 1. The volume mode diameter of the lipid globules of diet 1 was 0.5 um. Less than 10 vol.% had a diameter between 2 and 12 µm. The volume mode diameter of the lipid globules in diet 4 was 1.7 µm. More than 45 vol.% had a diameter between 2 and 12 µm.

**Table 2: Total Blood cholesterol levels and triglyceride (TG) levels (mmol/l) of rats on day 42 and 120 exposed to different diets from day 15 to day 42.**

| | | | Total cholesterol | TG |
|---|---|---|---|---|
| Diet 1 | Day 42 | Mean | 2.78 | 1.28 |
| | | S.E. | 0.13 | 0.37 |
| | Day 130 | Mean | 3.28 | 2.41 |
| | | S.E. | 0.36 | 0.41 |
| Diet 4 | Day 42 | Mean | 2.83 | 0.93 |
| | | S.E. | 0.18 | 0.11 |
| | Day 130 | Mean | 3.02 | 1.76 |
| | | S.E. | 0.46 | 0.35 |

Results are shown in Table 2. As can be seen at day 42, representing the direct diet effects, the differences on total blood cholesterol are very small. A small non significant increase with diet 4 was observed. This effect is probably less than the effect on cholesterol levels in human milk fed infants versus standard formula fed infants. At day 130, representing the programming, later in life effects, a much larger effect was observed, which were similar to that in experiment 1, being a reduced total blood cholesterol level. Blood triglyceride levels on the other hand were decreased both directly at day 42 but also later in life in rats having consumed diet 4, a diet of the present invention.

## Claims

1. A nutritional composition comprising lipid, wherein the lipid is present in the form of lipid globules, said lipid globules comprising a core comprising triglycerides derived from vegetable origin and a surface layer comprising cholesterol and phospholipids, for use in feeding a human subject having an age of 0 to 36 months, for use in
a) reducing blood cholesterol levels, and/or
b) reducing levels of blood LDL and/or VLDL,
when said human subject has reached an age above 36 months.

2. A nutritional composition comprising lipid, wherein the lipid is present in the form of lipid globules, said lipid globules comprising a core comprising triglycerides derived from vegetable origin and a surface layer comprising cholesterol and phospholipids, for use in feeding a human subject early in life, for use in
a) preventing hypercholesterolaemia and/or hyperlipoproteinemia, and/or
b) preventing atherosclerosis,
when said human subject has reached an age above 36 months.

3. The nutritional composition for use according to claim 1 or 2, wherein the lipid globules have a volume weighted mode diameter above 1.0 µm.

4. The nutritional composition for use according to claim 1, 2 or 3, wherein later in life is at least one year after feeding has stopped.

5. The nutritional composition for use according to any one of claims 1-4, wherein the nutritional composition comprises 10 to 50% lipid based on dry weight of the nutritional composition.

6. The nutritional composition for use according to any one of claims 1-5, wherein the nutritional composition comprises at least 0.02 wt.% cholesterol based on total lipid in the nutritional composition.

7. The nutritional composition for use according to any one of claims 1-6, wherein the nutritional composition comprises at least 0.5 wt.% of phospholipids based on total lipid in the nutritional composition.

8. The nutritional composition for use according to any one of claims 1-7, wherein at least 10 wt.% of the phospholipids is sphingomyelin.

9. The nutritional composition for use according to any one of claims 1-8, wherein the lipid in the nutritional composition has a fatty acid composition with a linoleic acid to alpha linolenic acid weight ratio of 2 to 7.

10. The nutritional composition for use according to any one of claims 1-9, wherein the nutritional composition further comprises docosahexaenoic acid, eicosapentaenoic acid and/or docosapentaenoic acid and the amount of the sum of docosahexaenoic acid, eicosapentaenoic acid and docosapentaenoic acid is at least 0.2 wt.% based on total fatty acids in the nutritional composition.

11. The nutritional composition for use according to any one of claims 1-10, wherein the cholesterol and phospholipids are of milk origin.

12. The nutritional composition for use according to any one of claims 1-11, wherein the nutritional composition comprises lipid, protein and digestible carbohydrates, wherein the lipid provides 30 to 60 % of the total calories, the digestible carbohydrates provide 30 to 80 % of the total calories and the protein provides 5 to 15 % of total calories.

13. The nutritional composition for use according to any one of claims 1-12, wherein the nutritional composition is in the form of a ready to drink liquid and comprises 50 to 80 kcal per 100 ml.

14. The nutritional composition for use according to any one of claims 1-13, wherein the nutritional composition is an infant formula or follow on formula.

15. The nutritional composition for use according to any one of claims 1-14, wherein the nutritional composition is a powder, suitable for reconstitution with water.

16. A non-medical method for
a) reducing blood cholesterol levels, and/or
b) reducing levels of blood LDL and/or VLDL,
in a human subject when said human subject has reached an age above 36 months,
by feeding a nutritional composition comprising lipid, wherein the lipid is present in the form of lipid globules, said lipid globules comprising a core comprising triglycerides derived from vegetable origin and a surface layer comprising cholesterol and phospholipids,
to the human subject having an age of 0 to 36 months.

## Patentansprüche

1. Nahrungszusammensetzung, die ein Lipid umfasst, wobei das Lipid in der Form von Lipidglobuli vorhanden ist, wobei die Lipidglobuli einen Kern, der aus pflanzlichen Quellen stammende Triglyceride umfasst, und eine Oberflächenschicht umfassen, die Cholesterin und Phospholipide umfasst, für die Verwendung in der Ernährung eines Menschen in einem Alter von 0 bis 36 Monaten, für die Verwendung in
a) der Verringerung der Cholesterinspiegel im Blut, und/oder
b) der Verringerung der LDL- und/oder VLDL-Spiegel im Blut,
wenn der Mensch ein Alter oberhalb 36 Monaten erreicht hat.

2. Nahrungszusammensetzung, die ein Lipid umfasst, wobei das Lipid in der Form von Lipidglobuli vorhanden ist, wobei die Lipidglobuli einen Kern, der aus pflanzlichen Quellen stammende Triglyceride umfasst, und eine Oberflächenschicht umfassen, die Cholesterin und Phospholipide umfasst, die Cholesterin und Phospholipide umfasst, für die Verwendung in der Ernährung eines Menschen im frühen Alter, für die Verwendung in
a) der Prävention von Hypercholesterinämie und/oder Hyperlipoproteinämie und/oder
b) der Prävention von Atherosklerose,
wenn der Mensch ein Alter oberhalb 36 Monaten erreicht hat.

3. Nahrungszusammensetzung für die Verwendung nach Anspruch 1 oder 2, wobei die Lipidglobuli einen Durchmesser im Volumen-gewichteten Modus oberhalb 1,0 µm aufweisen.

4. Nahrungszusammensetzung für die Verwendung nach Anspruch 1, 2 oder 3, wobei später im Leben wenigstens ein Jahr ist, nachdem die Ernährung beendet wurde.

5. Nahrungszusammensetzung für die Verwendung nach einem der Ansprüche 1 - 4, wobei die Nahrungszusammensetzung 10 bis 50% Lipid auf der Grundlage des Trockengewichts der Nahrungszusammensetzung umfasst.

6. Nahrungszusammensetzung für die Verwendung nach einem der Ansprüche 1 - 5, wobei die Nahrungszusammensetzung wenigstens 0,02 Gew.-% Cholesterin auf der Grundlage des Gesamtlipids in der Nahrungszusammensetzung umfasst.

7. Nahrungszusammensetzung für die Verwendung nach einem der Ansprüche 1 - 6, wobei die Nahrungszusammensetzung wenigstens 0,5 Gew.-% Phospholipide auf der Grundlage des Gesamtlipids in der Nahrungszusammensetzung umfasst.

8. Nahrungszusammensetzung für die Verwendung nach einem der Ansprüche 1 - 7, wobei wenigstens 10 Gew.-% der Phospholipide Sphingomyelin ist.

9. Nahrungszusammensetzung für die Verwendung nach einem der Ansprüche 1 - 8, wobei das Lipid in der Nahrungszusammensetzung eine Fettsäurezusammensetzung mit einem Gewichtsverhältnis von Linolsäure zu alpha-Linolensäure von 2 bis 7 aufweist.

10. Nahrungszusammensetzung für die Verwendung nach einem der Ansprüche 1 - 9, wobei die Nahrungszusammensetzung außerdem Docosahexaensäure, Eicosapentaensäure und/oder Docosapentaensäure umfasst, und die Menge der Summe von Docosahexaensäure, Eicosapentaensäure und Docosapentaensäure wenigstens 0,2 Gew.-% auf der Grundlage der Gesamtfettsäuren in der Nahrungszusammensetzung ist.

11. Nahrungszusammensetzung für die Verwendung nach einem der Ansprüche 1 - 10, wobei das Cholesterin und die Phospholipide aus Milch stammen.

12. Nahrungszusammensetzung für die Verwendung nach einem der Ansprüche 1 - 11, wobei die Nahrungszusammensetzung Lipid, Protein und verdauliche Kohlenhydrate umfasst, wobei das Lipid 30 bis 60% der Gesamtkalorien bereitstellt, die verdaulichen Kohlenhydrate 30 bis 80% der Gesamtkalorien bereitstellen, und das Protein 5 bis 15% der Gesamtkalorien bereitstellt.

13. Nahrungszusammensetzung für die Verwendung nach einem der Ansprüche 1 - 12, wobei die Nahrungszusammensetzung in der Form einer trinkfertigen Flüssigkeit ist und 50 bis 80 kcal pro 100 ml umfasst.

14. Nahrungszusammensetzung für die Verwendung nach einem der Ansprüche 1 - 13, wobei die Nahrungszusammensetzung eine Säuglingsanfangsnahrung oder eine Säuglingsfolgenahrung ist.

15. Nahrungszusammensetzung für die Verwendung nach einem der Ansprüche 1 - 14, wobei die Nahrungszusammensetzung ein Pulver ist, das für die Rekonstitution mit Wasser geeignet ist.

16. Nichtmedizinisches Verfahren für
a) die Verringerung der Cholesterinspiegeln im Blut, und/oder
b) die Verringerung der LDL- und/oder VLDL-Spiegel im Blut,
in einem Menschen wenn der Mensch ein Alter oberhalb 36 Monate erreicht hat,
durch Ernähren des Menschen in einem Alter von 0 bis 36 Monaten mit einer Nahrungszusammensetzung, die Lipid umfasst, wobei das Lipid in der Form von Lipidglobuli vorhanden ist, wobei die Lipidglobuli einen Kern, der aus einer pflanzlichen Quelle stammende Triglyceride umfasst, und eine Oberflächenschicht umfasst, die Cholesterin und Phospholipide umfasst.

## Revendications

1. Composition nutritionnelle comprenant des lipides, dans laquelle les lipides sont présents sous forme de globules gras, lesdits globules gras comprenant un noyau comprenant des triglycérides d'origine végétale et une couche superficielle comprenant du cholestérol et des phospholipides, destinée à être utilisée pour nourrir un sujet humain âgé de 0 à 36 mois, afin de
a) réduire les niveaux de cholestérol dans le sang, et/ou
b) réduire les niveaux de LDL et/ou VLDL dans le sang,
quand ledit sujet humain a dépassé l'âge de 36 mois.

2. Composition nutritionnelle comprenant des lipides, dans laquelle les lipides sont présents sous forme de globules gras, lesdits globules gras comprenant un noyau comprenant des triglycérides d'origine végétale et une couche superficielle comprenant du cholestérol et des phospholipides, destinée à être utilisée pour nourrir un sujet humain dans les premiers mois de sa vie, afin de
a) prévenir l'hypercholestérolémie et/ou l'hyperlipoprotéinémie, et/ou
b) prévenir l'artériosclérose,
quand ledit sujet humain a dépassé l'âge de 36 mois.

3. Composition nutritionnelle destinée à être utilisée selon la revendication 1 ou 2, dans laquelle les globules gras présentent un diamètre pondéré par le volume supérieur à 1,0 µm.

4. Composition nutritionnelle destinée à être utilisée selon la revendication 1, 2 ou 3, dans laquelle plus tard au cours de la vie signifie au moins un ans après l'arrêt de l'alimentation par ladite composition.

5. Composition nutritionnelle destinée à être utilisée selon l'une quelconque des revendications 1 à 4, dans laquelle la composition nutritionnelle comprend entre 10 et 50 % de lipides en poids sec de la composition nutritionnelle.

6. Composition nutritionnelle destinée à être utilisée selon l'une quelconque des revendications 1 à 5, dans laquelle la composition nutritionnelle comprend au moins 0,02 % en poids de cholestérol par rapport au poids total de lipides dans la composition nutritionnelle.

7. Composition nutritionnelle destinée à être utilisée selon l'une quelconque des revendications 1 à 6, dans laquelle la composition nutritionnelle comprend au moins 0,5 % en poids de phospholipides par rapport au poids total de lipides dans la composition nutritionnelle.

8. Composition nutritionnelle destinée à être utilisée selon l'une quelconque des revendications 1 à 7, dans laquelle au moins 10 % en poids des phospholipides sont la sphingomyéline.

9. Composition nutritionnelle destinée à être utilisée selon l'une quelconque des revendications 1 à 8, dans laquelle la composition en acides gras des lipides de la composition nutritionnelle présente un rapport massique d'acide linoléique et d'acide alpha linolénique compris entre 2 et 7.

10. Composition nutritionnelle destinée à être utilisée selon l'une quelconque des revendications 1 à 9, dans laquelle la composition nutritionnelle comprend en outre de l'acide docosahexaénoïque, de l'acide éicosapentaénoïque et/ou de l'acide docosapentaénoïque et la somme de l'acide docosahexaénoïque, de l'acide éicosapentaénoïque et de l'acide docosapentaénoïque représente au moins 0,2 % en poids du poids total d'acides gras dans la composition nutritionnelle.

11. Composition nutritionnelle destinée à être utilisée selon l'une quelconque des revendications 1 à 10, dans laquelle le cholestérol et les phospholipides proviennent du lait.

12. Composition nutritionnelle destinée à être utilisée selon l'une quelconque des revendications 1 à 11, dans laquelle la composition nutritionnelle comprend des lipides, des protéines et des glucides digestibles, dans laquelle les lipides apportent environ 30 à 60 % du total des calories, les glucides digestibles apportent environ 30 à 80 % du total des calories et les protéines apportent 5 à 15 % du total des calories.

13. Composition nutritionnelle destinée à être utilisée selon l'une quelconque des revendications 1 à 12, dans laquelle la composition nutritionnelle se présente sous la forme d'un liquide prêt à boire et comprend de 50 à 80 kcal pour 100 ml.

14. Composition nutritionnelle destinée à être utilisée selon l'une quelconque des revendications 1 à 13, dans laquelle la composition nutritionnelle est une préparation pour nourrissons ou une préparation de suite.

15. Composition nutritionnelle destinée à être utilisée selon l'une quelconque des revendications 1 à 14, dans laquelle la composition nutritionnelle est une poudre reconstituable dans l'eau.

16. Procédé non médical destiné à
a) réduire les niveaux de cholestérol dans le sang, et/ou
b) réduire les niveaux de LDL et/ou VLDL dans le sang,
chez un sujet humain quand ledit sujet humain a dépassé l'âge de 36 mois, consistant à nourrir d'une composition nutritionnelle comprenant des lipides, dans laquelle les lipides sont présents sous forme de globules gras, lesdits globules gras comprenant un noyau comprenant des triglycérides d'origine végétale et une couche superficielle comprenant du cholestérol et des phospholipides, le sujet humain âgé de 0 à 36 mois.
